# EUROPEAN PATENT SPECIFICATION

(11) **EP 1 720 900 B1**
(45) Date of publication and mention of the grant of the patent: **22.09.2010**
(21) Application number: 05708860.1
(22) Date of filing: 28.02.2005
(51) Int. Cl.: C07K 14/415, C12N 15/29, C12N 15/62, A61K 39/36

(54) **FUSION PROTEINS COMPRISING MODIFIED ALLERGENS OF THE NS-LTPS FAMILY, USE THEREOF AND PHARMACEUTICAL COMPOSITIONS COMPRISING THE SAME**
MODIFIZIERTE ALLERGENE DER NS-LTPS-FAMILIE ENTHALTENDE FUSIONSPROTEINE, DEREN VERWENDUNG UND PHARMAZEUTISCHE ZUSAMMENSETZUNG, DIE DIESE VERBINDUNGEN ENTHÄLT
PROTEINES DE FUSION CONTENANT DES ALLERGENES MODIFIES DE LA FAMILLE NS-LTPS, UTILISATION ET COMPOSITIONS PHARMACEUTIQUES LES RENFERMANT

(30) Priority: 27.02.2004 IT RM20040103
(43) Date of publication of application: 15.11.2006
(73) Proprietor: CONSIGLIO NAZIONALE DELLE RICERCHE, 00185 Roma (IT)
(72) Inventor: GERACI, Domenico, I-90149 Palermo (IT); BONURA, Angela, I-90147 Partinico PA (IT); COLOMBO, Paolo, I-90146 Palermo (IT)
(74) Representative: Germinario, Claudio
(86) International application number: PCT/IB2005/050714
(87) International publication number: WO 2005/085278

(56) References cited:
- WO-A-02/20790
- COSTA M A ET AL: "CDNA CLONING, EXPRESSION AND PRIMARY STRUCTURE OF PAR J I, A MAJOR ALLERGEN OF PARIETARIA JUDAICA POLLEN" FEBS LETTERS, ELSEVIER SCIENCE PUBLISHERS, AMSTERDAM, NL, vol. 341, no. 2/3, 21 March 1994 (1994-03-21), pages 182-186, XP002049655 ISSN: 0014-5793 cited in the application
- DURO G ET AL: "cDNA cloning, sequence analysis and allergological characterization of Par i 2.0101, a new major alergen of the Parietaria judaica pollen" FEBS LETTERS, ELSEVIER SCIENCE PUBLISHERS, AMSTERDAM, NL, vol. 399, 1996, pages 295-298, XP002191113 ISSN: 0014-5793 cited in the application
- COLOMBO P ET AL: "THE ALLERGENS OF PARIETARIA" INTERNATIONAL ARCHIVES OF ALLERGY AND IMMUNOLOGY, vol. 130, no. 3, March 2003 (2003-03), pages 173-179, XP009036928 ISSN: 1018-2438 cited in the application
- BONURA A ET AL: "HYPOALLERGENIC VARIANTS OF THE PARIETARIA JUDAICA MAJOR ALLERGEN PAR J 1: A MEMBER OF THE NON-SPECIFIC LIPID TRANSFER PROTEIN PLANT FAMILY" INTERNATIONAL ARCHIVES OF ALLERGY AND IMMUNOLOGY, vol. 126, no. 1, September 2001 (2001-09), pages 32-40, XP001037388 ISSN: 1018-2438
- MENNA T ET AL: "CHARACTERIZATION OF A DODECAPEPTIDE CONTAINING A DOMINANT EPITOPE OF PAR J 1 AND PAR 0 1, THE MAJOR ALLERGENS OF P. JUDAICA AND P. OFFICINALIS POLLEN" ALLERGY, MUNSKGAARD, COPENHAGEN, DK, vol. 54, no. 10, 1999, pages 1048-1057, XP009038929 ISSN: 0105-4538

### SEQUENCE LISTING

<110> Consiglio Nazionale delle Ricerche
<120> Fusion proteins
   <130> BW352R
   <160> 7
   <170> PatentIn version 3.2
<210> 1
   <211> 732
   <212> DNA
   <213> Parietaria judaica
   <222> (1)..(729)
<220>
   <221> misc_feature
   <222> (10)..(12); (40)..(42); (85)..(90); (148)..(150); (154)..(156); (271)..(273); (322)..(324); (352)..(354); (397)..(402); (460)..(462); (466)..(468); (535)..(537); (583)..(585)
   <223> n is a, c, g, or t
<400> 1
   gag gag gct nnn ggg aaa gtg gtg cag gat ata atg ccg nnn ctg cat      48
   Glu Glu Ala Xaa Gly Lys Val Val Gln Asp Ile Met Pro Xaa Leu His
   1               5                   10                  15
   ttc gtg aag ggg gag gag aag gag ccg tcg aag gag nnn nnn agc ggc      96
   Phe Val Lys Gly Glu Glu Lys Glu Pro Ser Lys Glu Xaa Xaa Ser Gly
               20                  25                  30
   acg aag aag ctg agc gag gag gtg aag acg acg gag cag aag agg gag      144
   Thr Lys Lys Leu Ser Glu Glu Val Lys Thr Thr Glu Gln Lys Arg Glu
           35                  40                  45
   gcc nnn aag nnn ata gtg cgc gcc acg aag ggc atc tcc ggt atc aaa      192
   Ala Xaa Lys Xaa Ile Val Arg Ala Thr Lys Gly Ile Ser Gly Ile Lys
       50                  55                  60
   aat gaa ctt gtc gcc gag gtc ccc aag aag nnn gat att aag acc act      240
   Asn Glu Leu Val Ala Glu Val Pro Lys Lys Xaa Asp Ile Lys Thr Thr
   65                  70                  75                  80
   ctc ccg ccc atc acc gcc gac ttc gac tgc nnn aag atc caa agt act      288
   Leu Pro Pro Ile Thr Ala Asp Phe Asp Cys Xaa Lys Ile Gln Ser Thr
                   85                  90                  95
   att ttc aga ggt tac tat gga ttc caa gaa acc nnn ggg act atg gtg      336
   Ile Phe Arg Gly Tyr Tyr Gly Phe Gln Glu Thr Xaa Gly Thr Met Val
               100                 105                 110
   aga gcg ctg atg ccg nnn ctg ccg ttc gtg cag ggg aaa gag aaa gag 384
   Arg Ala Leu Met Pro Xaa Leu Pro Phe Val Gln Gly Lys Glu Lys Glu
   115 120 125
   ccg tca aag ggg nnn nnn agc ggc gcc aaa aga ttg gac ggg gag acg 432
   Pro Ser Lys Gly Xaa Xaa Ser Gly Ala Lys Arg Leu Asp Gly Glu Thr
   130 135 140
   aag acg ggg ccg cag agg gtg cac gct nnn gag nnn atc cag acc gcc 480
   Lys Thr Gly Pro Gln Arg Val His Ala Xaa Glu Xaa Ile Gln Thr Ala
   145 150 155 160
   atg aag act tat tcc gac atc gac ggg aaa ctc gtc agc gag gtc ccc 528
   Met Lys Thr Tyr Ser Asp Ile Asp Gly Lys Leu Val Ser Glu Val Pro
   165 170 175
   aag cac nnn ggc atc gtt gac agc aag ctc ccg ccc att gac gtc aac 576
   Lys His Xaa Gly Ile Val Asp Ser Lys Leu Pro Pro Ile Asp Val Asn
   180 185 190
   atg gac nnn aag aca gtt gga gtg gtt cct cgg caa ccc caa ctt cca 624
   Met Asp Xaa Lys Thr Val Gly Val Val Pro Arg Gln Pro Gln Leu Pro
   195 200 205
   gtc tct ctc cgt cat ggt ccc gtc acg ggc cca agt gat ccc gcc cac 672
   Val Ser Leu Arg His Gly Pro Val Thr Gly Pro Ser Asp Pro Ala His
   210 215 220
   aaa gca cgg ttg gag aga ccc cag att aga gtt ccg ccc ccc gca ccg 720
   Lys Ala Arg Leu Glu Arg Pro Gln Ile Arg Val Pro Pro Pro Ala Pro
   225 230 235 240
   gaa aaa gcc taa 732
   Glu Lys Ala
<210> 2
   <211> 243
   <212> PRT
   <213> Parietaria judaica
<220>
   <221> misc_feature
   <222> (4)..(4)
   <223> The 'Xaa' at location 4, 14, 29, 30, 50, 52, 75, 91, 108, 118, 133, 154, 156, 179, 195 stands for Asn, Ser, Thr, lie,Met, Gly, Ala, Val, Gln or Leu.
<400> 2
   Glu Glu Ala Xaa Gly Lys Val Val Gln Asp Ile Met Pro Xaa Leu His
   1 5 10 15
   Phe Val Lys Gly Glu Glu Lys Glu Pro Ser Lys Glu Xaa Xaa Ser Gly
   20 25 30
   Thr Lys Lys Leu Ser Glu Glu Val Lys Thr Thr Glu Gln Lys Arg Glu
   35 40 45
   Ala Xaa Lys Xaa Ile Val Arg Ala Thr Lys Gly Ile Ser Gly Ile Lys
   50 55 60
   Asn Glu Leu Val Ala Glu Val Pro Lys Lys Xaa Asp Ile Lys Thr Thr
   65 70 75 80
   Leu Pro Pro Ile Thr Ala Asp Phe Asp Cys Xaa Lys Ile Gln Ser Thr
   85 90 95
   Ile Phe Arg Gly Tyr Tyr Gly Phe Gln Glu Thr Xaa Gly Thr Met Val
   100 105 110
   Arg Ala Leu Met Pro Xaa Leu Pro Phe Val Gln Gly Lys Glu Lys Glu
   115 120 125
   Pro Ser Lys Gly Xaa Xaa Ser Gly Ala Lys Arg Leu Asp Gly Glu Thr
   130 135 140
   Lys Thr Gly Pro Gln Arg Val His Ala Xaa Glu Xaa Ile Gln Thr Ala
   145 150 155 160
   Met Lys Thr Tyr Ser Asp Ile Asp Gly Lys Leu Val Ser Glu Val Pro
   165 170 175
   Lys His Xaa Gly Ile Val Asp Ser Lys Leu Pro Pro Ile Asp Val Asn
   180 185 190
   Met Asp Xaa Lys Thr Val Gly Val Val Pro Arg Gln Pro Gln Leu Pro
   195 200 205
   Val Ser Leu Arg His Gly Pro Val Thr Gly Pro Ser Asp Pro Ala His
   210 215 220
   Lys Ala Arg Leu Glu Arg Pro Gln Ile Arg Val Pro Pro Pro Ala Pro
   225 230 235 240
   Glu Lys Ala
<210> 3
   <211> 732
   <212> DNA
   <213> Parietaria judaica
   <221> CDS
   <222> (1)..(729)
<400> 3
   gag gag gct agc ggg aaa gtg gtg cag gat ata atg ccg tgc ctg cat      48
   Glu Glu Ala Ser Gly Lys Val Val Gln Asp Ile Met Pro Cys Leu His
   1               5                   10                  15
   ttc gtg aag ggg gag gag aag gag ccg tcg aag gag agc agc agc ggc      96
   Phe Val Lys Gly Glu Glu Lys Glu Pro Ser Lys Glu Ser Ser Ser Gly
               20                  25                  30
   acg aag aag ctg agc gag gag gtg aag acg acg gag cag aag agg gag     144
   Thr Lys Lys Leu Ser Glu Glu Val Lys Thr Thr Glu Gln Lys Arg Glu
           35                  40                  45
   gcc tgc aag tgc ata gtg cgc gcc acg aag ggc atc tcc ggt atc aaa     192
   Ala Cys Lys Cys Ile Val Arg Ala Thr Lys Gly Ile Ser Gly Ile Lys
       50                  55                  60
   aat gaa ctt gtc gcc gag gtc ccc aag aag tgc gat att aag acc act     240
   Asn Glu Leu Val Ala Glu Val Pro Lys Lys Cys Asp Ile Lys Thr Thr
   65                  70                  75                  80
   ctc ccg ccc atc acc gcc gac ttc gac tgc tcc aag atc caa agt act     288
   Leu Pro Pro Ile Thr Ala Asp Phe Asp Cys Ser Lys Ile Gln Ser Thr
                   85                  90                  95
   att ttc aga ggt tac tat gga ttc caa gaa acc agc ggg act atg gtg     336
   Ile Phe Arg Gly Tyr Tyr Gly Phe Gln Glu Thr Ser Gly Thr Met Val
               100                 105                 110
   aga gcg ctg atg ccg tgc ctg ccg ttc gtg cag ggg aaa gag aaa gag     384
   Arg Ala Leu Met Pro Cys Leu Pro Phe Val Gln Gly Lys Glu Lys Glu
           115                 120                 125
   ccg tca aag ggg agc agc agc ggc gcc aaa aga ttg gac ggg gag acg     432
   Pro Ser Lys Gly Ser Ser Ser Gly Ala Lys Arg Leu Asp Gly Glu Thr
       130                 135                 140
   aag acg ggg ccg cag agg gtg cac gct tgt gag tgc atc cag acc gcc     480
   Lys Thr Gly Pro Gln Arg Val His Ala Cys Glu Cys Ile Gln Thr Ala
   145                 150                 155                 160
   atg aag act tat tcc gac atc gac ggg aaa ctc gtc agc gag gtc ccc     528
   Met Lys Thr Tyr Ser Asp Ile Asp Gly Lys Leu Val Ser Glu Val Pro
                   165                 170                 175
   aag cac tgc ggc atc gtt gac agc aag ctc ccg ccc att gac gtc aac 576
   Lys His Cys Gly Ile Val Asp Ser Lys Leu Pro Pro Ile Asp Val Asn
   180 185 190
   atg gac tgc aag aca gtt gga gtg gtt cct cgg caa ccc caa ctt cca 624
   Met Asp Cys Lys Thr Val Gly Val Val Pro Arg Gln Pro Gln Leu Pro
   195 200 205
   gtc tct ctc cgt cat ggt ccc gtc acg ggc cca agt gat ccc gcc cac 672
   Val Ser Leu Arg His Gly Pro Val Thr Gly Pro Ser Asp Pro Ala His
   210 215 220
   aaa gca cgg ttg gag aga ccc cag att aga gtt ccg ccc ccc gca ccg 720
   Lys Ala Arg Leu Glu Arg Pro Gln Ile Arg Val Pro Pro Pro Ala Pro
   225 230 235 240
   gaa aaa gcc taa 732
   Glu Lys Ala
<210> 4
   <211> 243
   <212> PRT
   <213> Parietaria judaica
<400> 4
   Glu Glu Ala Ser Gly Lys Val Val Gln Asp Ile Met Pro Cys Leu His
   1 5 10 15
   Phe Val Lys Gly Glu Glu Lys Glu Pro Ser Lys Glu Ser Ser Ser Gly
   20 25 30
   Thr Lys Lys Leu Ser Glu Glu Val Lys Thr Thr Glu Gln Lys Arg Glu
   35 40 45
   Ala Cys Lys Cys Ile Val Arg Ala Thr Lys Gly Ile Ser Gly Ile Lys
   50 55 60
   Asn Glu Leu Val Ala Glu Val Pro Lys Lys Cys Asp Ile Lys Thr Thr
   65 70 75 80
   Leu Pro Pro Ile Thr Ala Asp Phe Asp Cys Ser Lys Ile Gln Ser Thr
   85 90 95
   Ile Phe Arg Gly Tyr Tyr Gly Phe Gln Glu Thr Ser Gly Thr Met Val
   100 105 110
   Arg Ala Leu Met Pro Cys Leu Pro Phe Val Gln Gly Lys Glu Lys Glu
   115 120 125
   Pro Ser Lys Gly Ser Ser Ser Gly Ala Lys Arg Leu Asp Gly Glu Thr
   130 135 140
   Lys Thr Gly Pro Gln Arg Val His Ala Cys Glu Cys Ile Gln Thr Ala
   145 150 155 160
   Met Lys Thr Tyr Ser Asp Ile Asp Gly Lys Leu Val Ser Glu Val Pro
   165 170 175
   Lys His Cys Gly Ile Val Asp Ser Lys Leu Pro Pro Ile Asp Val Asn
   180 185 190
   Met Asp Cys Lys Thr Val Gly Val Val Pro Arg Gln Pro Gln Leu Pro
   195 200 205
   Val Ser Leu Arg His Gly Pro Val Thr Gly Pro Ser Asp Pro Ala His
   210 215 220
   Lys Ala Arg Leu Glu Arg Pro Gln Ile Arg Val Pro Pro Pro Ala Pro
   225 230 235 240
   Glu Lys Ala
<210> 5
   <211> 18
   <212> DNA
   <213> Artificial
<220>
   <223> forward primer for insertion of mutation in position 29 and 30
<400> 5
   gagagcagca gcggcagc 18
<210> 6
   <211> 30
   <212> DNA
   <213> Artificial
<220>
   <223> forward primer for insertion of mutation in position 4
<400> 6
   gtgggatccg aggaggctag cgggaaagtg 30
<210> 7
   <211> 24
   <212> DNA
   <213> Artificial
<220>
   <223> reverse parj2 primer
<400> 7
   gggggatcca tagtaacctc tgaa 24

## Description

### Field of the invention

The present invention lies within the fields of the prevention and the treatment of allergic symptoms associated to allergens belonging to the non-specific Lipid Transfer Protein (ns-LTPs) family.

### State of the Art

ns-LTPs proteins are small proteic molecules of approximately 10 KDa that demonstrate high stability, and are naturally present in all vegetal organisms studied to date. These proteins are characterised by their ability to transport lipids through membranes *in vitro.*

In several species they have also been identified as allergens, as in the case of the Rosaceae Prunoideae (peach, apricot, plum) and Pomoideae (apple), as in the Urticacee like Parietaria. The genus *Parietaria* includes 5 species, *P. Judaica* being the most allergenic one.

The allergic reaction, also called Type I hypersensitivity, is induced by an IgE-mediated response to environmental antigens, usually innocuous and present, e.g., in pollen grains. The IgE/Allergens interaction is the initial event of a cascade of reactions leading to the release of mediators, like histamine, responsible for the allergic symptomatology. When localised at skin level, the release of histamine causes itch, erythema and edema, whereas when generalised it causes bronchoconstriction at the brochopulmonary level and impressive phenomena involving the cardiovascular system.

The most common allergic illnesses are rhinitis, conjunctivitis, urticaria, angioedema, eczema, dermatitides, asthma and, in extreme cases, anaphylactic shock.

Recombinant DNA technology allowed the isolation of various allergens of the ns-LTPs family, among them of the major allergens of *Parietaria* denominated Parj1 and Parj2 (Colombo, P., et al., The allergens of Parietaria Int Arch Allergy Immunol. 2003 Mar;130(3):173-9, Review).

Parj1 is a protein of 176 amino acids and a molecular weight of 18.450 Da. The N-terminal sequence exhibits an amino acid composition characteristic of signal sequences of glycosilated proteins. The mature protein is composed of 139 amino acids, with a molecular weight of 14726 Da. It is a major allergen, since it binds more than 90% of sera of subjects allergic to *Parietaria judaica.* (Costa et al, cDNA cloning, expression and primary structure of Par jI, a major allergen of Parietaria judaica pollen. FEBS Lett., 1994 Mar 21; 341 (2-3) :182-6.)

Parj2 is a protein of 133 amino acids, and it contains a signal peptide of 31 amino acids. The mature protein is composed of 102 amino acids, with a molecular weight of 11344 Da. It exhibits a 45% homology with the Parj1 at the amino acid level and is it also a major allergen, since it reacts with the near-totality of the sera of allergic subjects (Duro, G., et al., cDNA cloning, sequence analysis and allergological characterization of Parj 2.0101, a new major allergen of the Parietaria Judaica pollen. FEBS Lett, 1996. 399(3): p. 295-8).

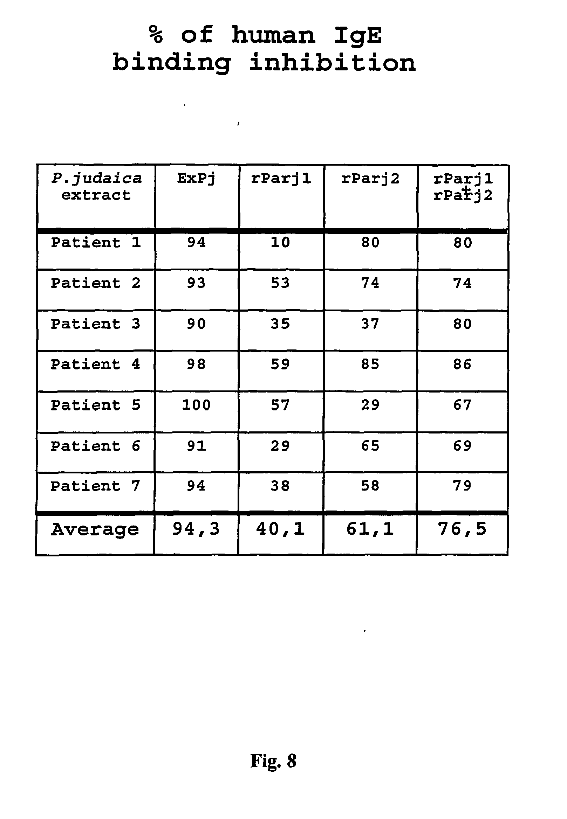

In spite of the their structural homology, Parj1 and Parj2 are anyhow two independent allergens containing epitopes them also independent, as highlighted by cross-inhibition experiments. When a pool of sera of allergic subjects is preincubated with the recombinant Parj1 and Parj2 allergens, IgE binding to the 10-14 kDa region is totally inhibited, suggesting that only these two allergens are present in this region and that together they are capable of inhibiting the majority of specific IgE against *Parietaria judaica* allergens (Table Fig 8).

The Parj1 and Parj2 allergens exhibit all the characteristics of the ns-LTP and the structural model of the Parj1 was determined using the crystal of the ns-LTP from soy seed as reference. According to said model, both molecules have 4 disulphide bridges, in the order: 4-52, 14-29, 30-75, 50-91. All ns-LTP proteins considered, when suitably aligned as illustrated in Fig. 2 of Pat. App. WO-A-02/20790, contain four disulphide bridges between cysteine residues in positions corresponding to the aboveindicated positions of Parj1 or Parj2.

By applying a strategy of site-specific mutagenesis, it has previously been demonstrated the relevance of the disulphide bridges in the formation of the IgE epitopes (WO-A-02/20790) and the existence of a dominant epitope in the loop1 region comprised between amino acids 1 and 30. (Colombo, P., et al., Identification of an immunodominant IgE epitope of the Parietaria Judaica major allergen. J. Immunol, 1998. 160(6): p. 2780-5).

From a therapeutic standpoint, various pharmacological treatments of the allergic symptomatology do exist, whereas the sole preventive therapy is represented by the specific immunotherapy (SIT). This therapy foresees the subcutaneous administration of diluted quantities of allergen to the patient so as to suppress the specific reaction towards the allergen. However, the majority of the commercial protein extracts used therefor are anyhow crude extracts, mixtures of several components in which a precise standardization of the allergenic component is difficult. Thus, the SIT strategy can entail the administration of allergenic components towards which the patient is not sensitive, inducing the production of IgEs specific towards other components of the extract. Moreover, the administration of the total allergen entails the risk of side effects, which could even cause anaphylactic shock. In order to eliminate some of the disadvantages described hereto, there have been developed alternative molecules with reduced side effects, i.e., capable of not interacting with the IgEs while maintaining the capability of immunosuppressing the T response and capable of stimulating the production of IgG immunoglobulins.

The preceding work of the present Authors, disclosed in Pat. App. WO-A-02/20790, lies within the same purview. By genetic manipulation, there were produced variant forms, or muteins, of ns-LTP allergens, characterised by the partial or total elimination of the disulphide bridges typical of the native protein. These muteins have a reduced allergenic activity with characteristics such as to make them useful in SIT as molecules substituting the native proteins.

A different approach is that described by B. Linhart in Pat. App. EP-A-1 219 301. In this case, there are described hybrid polypeptides containing at least two wild type allergenic proteins or fragments thereof. Working on Phlenum pratense pollen allergens Phl-p or on parasite allergens Der, Linhart observes a reduced allergenicity of the hybrid peptides.

However, there remains a need for novel tools suitable for the treatment of specific allergic forms, like those caused by the ns-LTPs proteins, tools combining the characteristics of reduced allergenicity with high immunotherapeutic effectiveness and easy accessibility with regard to both their preparation and their use. Scope of the present invention is to satisfy this need.

### Summary of the Invention

Epidemiological studies demonstrated that subjects generally allergic to various plants belonging to the same genus, or to the same species, or even to a single specific plant variety, in the majority of cases have IgEs against plural allergens produced by the plant. E.g., in the case of subjects allergic to *Parieta ria judaica,* those usually have IgEs against both of the major allergens, i.e., the proteins Parj1 and Parj2. As highlighted in Figure 8 (Table) in a binding inhibition assay between the human IgEs in sera of PJ allergic patients and an extract of P. judaica, the percentage of inhibition induced by the mixture of wild type Parj1 and Parj2 is usually higher than that induced by the individual allergens. Hence, any one therapeutic formulation suitable for the treatment of allergy should comprise all the main allergens, or muteins thereof, produced by one or more plants responsible for the allergy.

The present invention is based on the unexpected discovery that hybrid proteins, obtained by the fusion of the polypeptide sequences of plural allergens in mutated form, have characteristics that are advantageous both from the therapeutic and preparative standpoint, as well as with regard to the management of the medicament compared to mere mixtures of plural allergens.

Main object of the present invention are fusion proteins comprising amino acid sequences of the different allergens Parj1 and Parj2, belonging to the ns-LTPs protein family, wherein said sequences lack one or more of the four disulphide bridges present in the sequence of the wild type allergens, in particular lacking at least one disulphide bridge in the amino-terminal region comprised between the amino acid residues 1 and 30. The amino acid sequence of each of the allergens is independently mutated by elimination or substitution of one or more cysteine residues involved in the formation of a disulphide bridge, though maintaining essentially the same length of the sequences of wild type allergens.

The allergens of the invention are produced by plants belonging to the same genus, or to the same species, or preferably to the same vegetal variety. In a preferred embodiment the fusion protein is a heterodimer protein comprising the amino acid sequences of two different allergens, e.g., allergens of the same plant like the allergens Parj1 and Parj2 of the *Parietaria Judaica* species.

In this case, the amino acid sequences of Parj1 and Parj2 allergens will both be independently modified by substitution of cysteine residues with residues not capable of forming a disulphide bridge in positions 29 and 30 or 4, 29 and 30 or 29, 30, 50, 52.

Further objects of the invention are a nucleotide sequence comprising the DNA coding for the fusion protein of the invention, an expression or cloning system comprising the nucleotide sequence at issue flanked by suitable sequences for controlling, promoting and regulating the expression, and a host cell transformed by means of said expression or cloning system.

Other objects of the invention are medical uses of the fusion protein, in particular as hypoallergenic immunological agent in the specific immunotherapy (SIT) treatment of allergies, as well as pharmaceutical compositions comprising the fusion protein and a pharmaceutically acceptable excipient.

Further objects of the invention are methods of preparation of the fusion protein in which suitably mutated amino acid sequences of different allergens are produced and bound directly or via a linker for chemical synthesis or by expression, in the form of fusion protein, in genetically modified host cells, as well as methods of preparation of the pharmaceutical compositions.

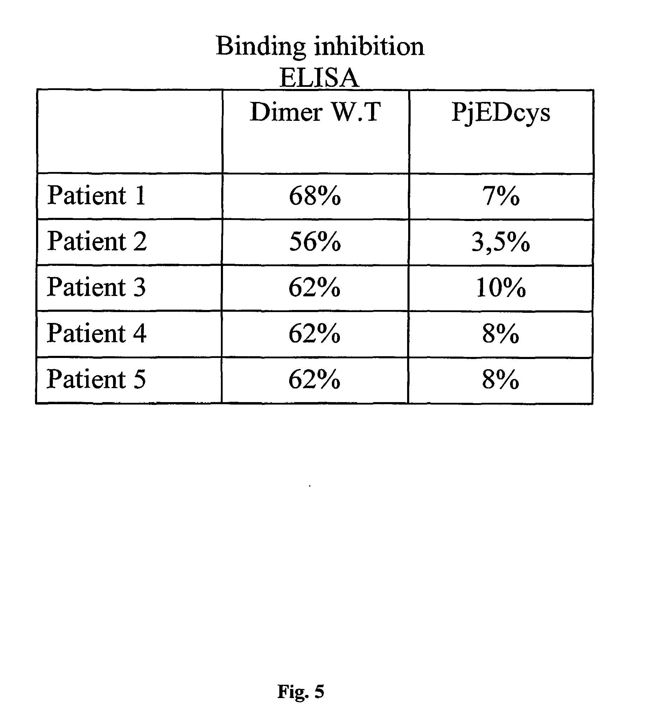

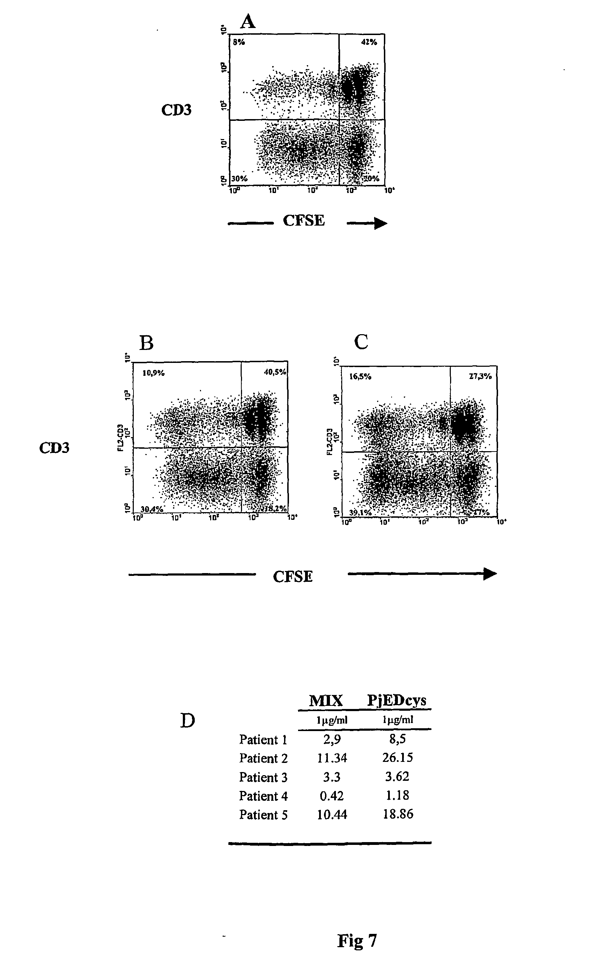

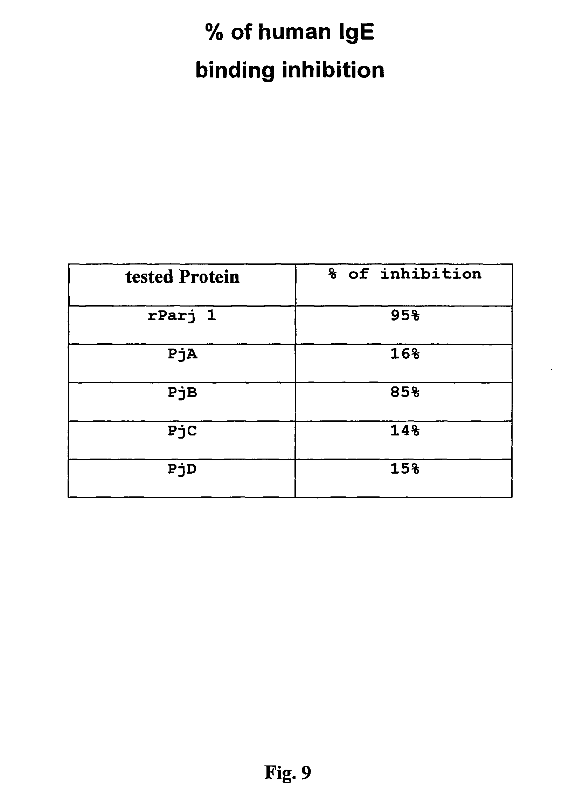

The fusion molecules according to the invention provide indisputable advantages. First of all, they exhibit a capability of interacting with the IgEs that is markedly reduced with respect to: the individual allergens or mixtures of wild type allergens, as it is apparent from a comparison of the data reported in the Table of Fig.5 (PjEDcys lane) to those in the Table of Fig. 8; the individual modified allergens, as it is apparent from a comparison of the Table of Fig. 5 with the Table reported in Fig. 9; or to the heterodimer of wild type (Wt) allergens, as again apparent from the Table of Fig. 5. Moreover, cytofluorometric analysis of peripheral blood cells CD3+ proliferation demonstrates that this reduced allergenicity is advantageously accompanied by an unaltered or even enhanced immunogenic capability, as illustrated in Fig. 7, panels B and C, and as resumed by the Table in the same Fig. 7. Not only the reduction of the allergenic characteristics of the heterodimer, accompanied by a marked immunogenic capability with respect to the individual wild type or mutated allergens or to mixtures thereof, was unforeseeable; already the mere maintenance by the hybrid protein of the typical features of the individual modified allergen was a characteristic in no way expectable.

Moreover, with respect to the mere mixture of allergens, or of muteins thereof, the heterodimers of the invention entail the further advantage of being producible via a single process, of course simplifying all the procedures of production, control, storage, authorization to sale and use, with an indisputable saving of times and material and financial means.

### Brief description of the figures

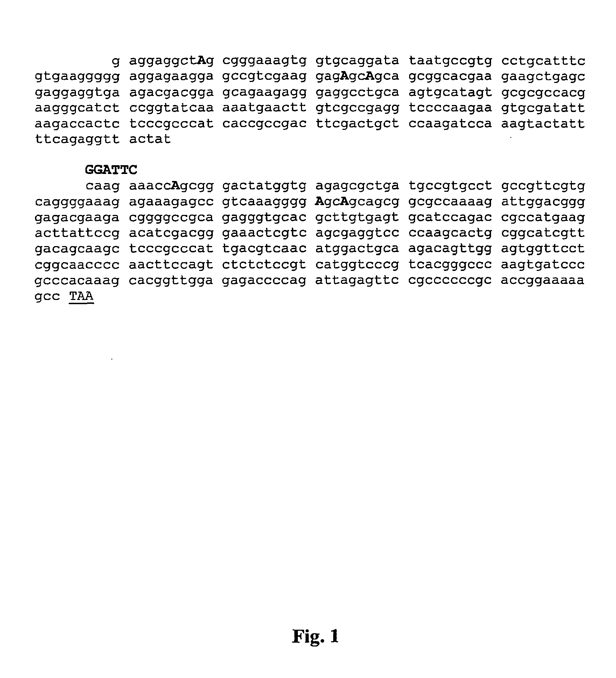

Figure 1: Nucleotide sequence of the Parj2-Parj1 dimer in its form mutated on the residues in positions 10, 85, 88, 122, 397 and 400. In all the positions indicated the nucleotide T was substituted with the nucleotide A (in bold type). The "GGATTC" spacer between the sequences coding for the two allergens Parj2 and Parj1 is also highlighted in bold type (SEQ ID NO:3)

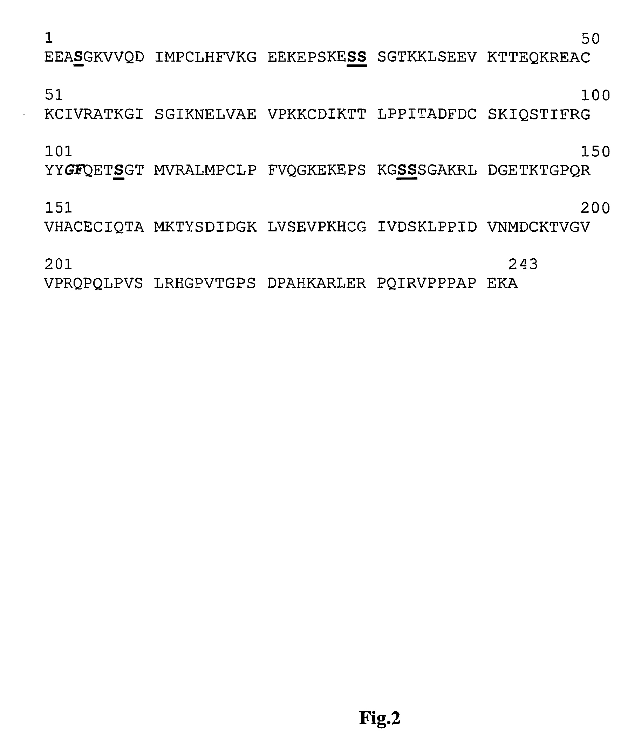

Figure 2: Amino acid sequence of the Parj2-Parj1 heterodimer in its form mutated on the residues in positions 4, 29 and 30 in each of the two molecules (PjEDcys). The amino acid sequence is expressed in one-letter code. Underlined amino acids indicate the substitutions effected. Amino acids in bold type and italics indicate the amino acids glycine and phenylalanine introduced with the cloning. (SEQ ID NO 4)

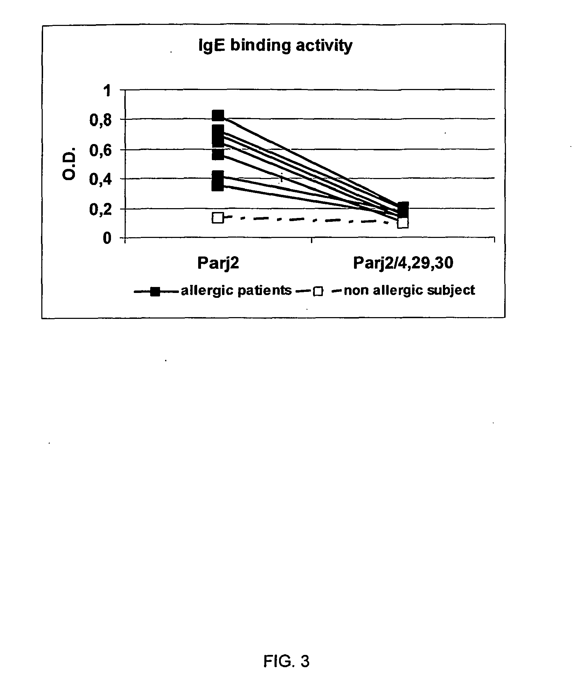

Figure 3: ELISA detection of the binding capability of the Parj2 allergen as compared to the binding activity of the Parj2/4,29,30 mutant. Lines with black squares indicate the sera of Pj allergic subjects, the line with white squares indicates a sera of a Pj non allergic patient.

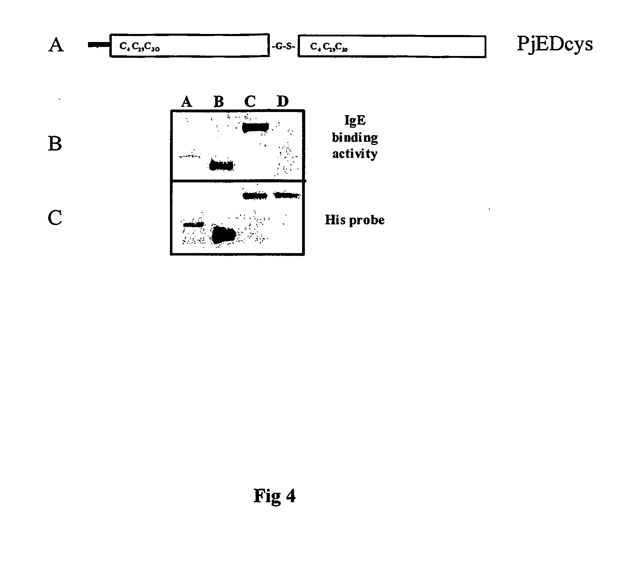

Figure 4: Panel A: schematic representation of the PjEDcys mutant. Panel B: Western blot Analysis carried out by using the recombinant Parj1 proteins (lane A), Parj2 (lane B), Parj2-Parj1 dimer (Wt dimer clone) (lane C), PjEDcys (lane D) with a serum of Pj allergic subject. Panel C: the same membrane of Panel B incubated with a probe specific for the histidine tail.
Figure 5: ELISA detection of human IgEs binding inhibition by the Wt dimer and PjEDcys recombinant proteins, using a *Parietaria judaica* crude extract as antigen and 5 sera from Pj allergic patients.

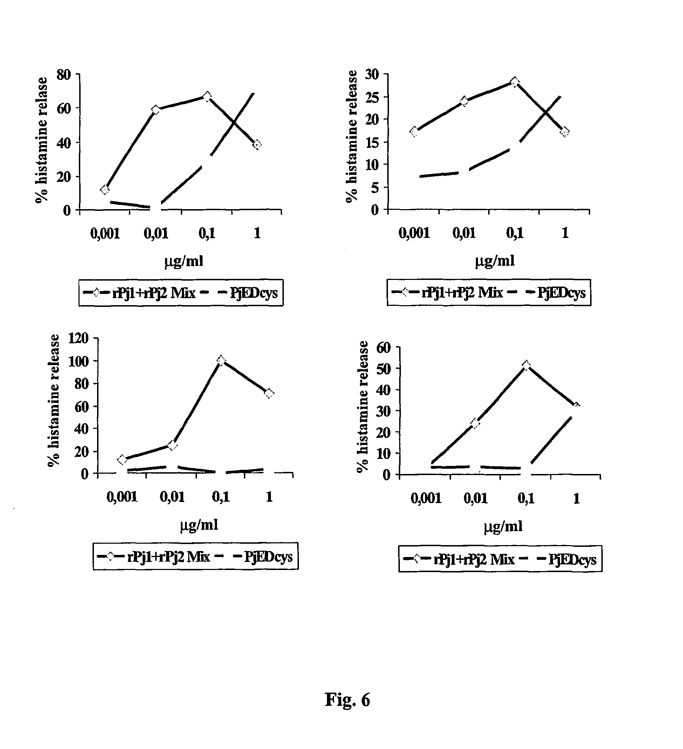

Figure 6: Testing of histamine release from blood of Pj allergic patients. The antigens used were: an equimolar mixture of the two rParj1 and rPar2 allergens (line with rhombs, denominated rPj1+rPj2) and the PjEDcys mutant (line with squares). On the x-axis the amounts of protein used, and on the y-axis the percentage of histamine released with respect to the percentage of total histamine of the patient's mastocytes are reported.
Figure 7: Cytofluorometric analysis of the proliferation of CD3+ cells from PBMC.
   CSFE-labeled cells were stimulated with a solution containing a mixture of the Parj1 and Parj2 allergens (panel B) and a mixture containing an equimolar amount of the PjEDcys hybrid (panel C). Panel A shows the unstimulated control culture.
   The bottom table resumes the percentage of CD3+ cells capable of proliferating after antigenic stimulation in 5 allergic subjects. Numbers indicate the percentages of stimulation subtracted of the unstimulated cells.
Figure 8: The table reports the results of an ELISA test of human IgEs binding inhibition by individual Wt allergens or mixture thereof.
Figure 9: The table reports the results of an ELISA test of human IgEs binding inhibition by the mutated allergens PjA, PjB, PjC and PjD described in WO-A-02/020790.

### Detailed description of the invention

The peptide sequences of wild type ns-LTPs allergens produced by various plants, like Parietaria judaica (Parj1 and Parj2), soy, lyces, ricco, tobacco, orysa, mais, spiol and wheat are reported in Pat. App. WO-A-02/20790. All molecules have four disulphide bridges between eight cysteine residues in highly conserved positions corresponding, when suitably aligned, to positions 4, 14, 29, 30 50, 52, 75 and 91, of the Parj1 and Parj2 molecules. The cysteine residues involved in disulphide bridges are the couplings 4-51, 14-29, 30-75 and 50-91.

Muteins of these allergens with a reduced capability of forming disulphide bridges are molecules in which one or more cysteine residues involved in the -SS- bond have been eliminated or substituted with other residues not capable of taking part in the binding, yet without sterically altering the spatial conformation of the molecule, e.g., Asn, Ser, Thr, Ile, Met, Gly, Ala, Val, Gln or Leu. Preferred muteins are obtained by elimination of two, three or four disulphide bridges; e.g., those corresponding to bonds 14-29 and/or 30-75 and/or 4-51 and/or 50-91 of the Parj1 or Parj2 molecule. Molecules mutated by substitution of the cysteine residue with a serine or alanine residue in positions 29, 30, or 4, 29, 30, or 29, 30, 50, 52 of Parj1 or Parj2, or in the corresponding positions of the other ns-LTPs allergens, exhibit the best properties of low allergenicity. Apart from the above ones, the muteins used in the invention exhibit no other modifications, and therefore maintain substantially unaltered the sequence, the length and the molecular weight of the wild type allergen.

The polynucleotide sequences coding for wild type allergens of the ns-LTPs family are available to the public on databanks like EMBL, or described in the state of the art. In particular, the nucleotide sequences of *Parietaria judaica* allergens are described in the abovementioned WO-A-02/20790 and in Duro, G., et al FEBS Letter 1996 (*supra*).

The preparation of the muteins of the invention is carried out via any known method suitable for the introduction of variations on individual amino acid residues along the polypeptide sequence of proteins. Usually, the variation is conducted via a site-specific point mutation at the level of the coding nucleotide sequence by DNA PCR method and using suitable synthetic oligonucleotides. The procedures are described, e.g., in the preceding application WO-A-02/20790.

The fusion proteins of the invention contain the amino acid sequences, suitably mutated, of allergens obtained from the same vegetal family, e.g., from fagaceae, urticaceae, oleaceae, composites or graminae; or from the same genus, e.g., *Parietaria*; or from the same species, e.g., *P. judaica, officinalis* or *lusitanica*; or, even better, from the same plant variety. Preferred fusion proteins are those comprising muteins of the main allergens of the *Parietaria judaica,* i.e., Parj1 and Parj2 or isoforms thereof known and deposited, e.g., in EMBL. The two proteins bound in an heterodimer can be modified according to the same scheme or to different schemes. Thus, the two proteins could contain disulphide bridges differing the one from the other in number and/or position. Preferred embodiments of the invention provide allergens individually and independently mutated in one or more of the positions corresponding to positions 4, 29 and 30 of the amino acid sequence of the major allergens of P. *judaica.* The fusion protein of the invention contains, e.g., the Parj1 and Parj2 allergens modified both in positions 29 and 30 or 4, 29 and 30 or 29, 30, 50, 52.

The preparation of the hybrid molecule occurs by fusion of the two protein portions corresponding to the two allergens. Forming by synthesis a direct chemical bond e.g., peptidic, between the N-terminal and C-terminal residues of the two portions is viable; yet, the method used preferably implies the construction of a polynucleotide molecule coding for the allergenic proteins in fused form and suitably mutated in the desired positions (SEQ ID NO:1).

The two portions constituting the resulting heterodimer can be directly bound or separated by one or more amino acid residues. According to a scheme well-known to a person skilled in the art and detailed in the examples, clones containing the material coding for the suitably mutated individual allergenic proteins are amplified, digested with restriction enzymes and the coding fragments are bound and integrated into expression vectors. In order to facilitate its purification, the hybrid protein can optionally be expressed as fusion protein with a binding molecule exhibiting a specific affinity for a determined partner molecule; e.g., with a histidine tail in the amino terminal region allowing the purification on a His-trap column.

The cloning and expression systems used for the preparation of the fusion protein can be vectors suitable for prokaryotic or eukaryotic cells, e.g. the commercial pQE30 prokaryotic vector.

Pharmaceutical compositions suitable in the administration of the molecules of the invention are compositions in the form of aqueous, hydroalcoholic or oily solutions, of emulsions or suspensions, in aqueous or oily medium, or of liposome suspensions. Advantageously, the composition is formulated to attain a delayed release. Therefor, oily media or media containing suitable thickeners may be used. Besides the described formulations in liquid form, the compositions of the invention can be in semi-solid form like creams, pomades, gels or other forms suitable for topical application. Implants for subcutaneous application aimed at a prolonged release may be used as well. In this case the molecules of the invention are incorporated into a biodegradable or biodispersible polymer matrix under the action of the patient's natural enzyme system. Therefor, polymers such as polylactate or polyglycolate or polylactate/glycolate copolymers will be used.

The compositions at issue are formulated for a parenteral administration, in subcutaneous, intramuscular or intravenous use, for a topical administration on the skin or mucosae or for oral administration.

The fusion molecules of the invention are characterised by a marked hypoallergenicity with respect to the individual monomer allergens (Fig. 4, panel B and C) or with respect to a heterodimer molecule consisting of the Wt allergens (Fig. 5).

The hypoallergenic molecules of the invention find valid use as medicaments in the preventive and curative treatment of allergies caused by plural plant allergens, and in particular as desensitizers or immunosuppressants with reduced anaphylactic activity in SIT treatments. Exemplary allergic diseases that may be treated with the fusion protein of the invention are rhinitis, conjunctivitis, urticaria, angioedema, eczema, dermatitides, asthma, anaphylactic shock.

Lastly, the hypoallergenic fusion proteins of the invention find use for the preparation of DNA vaccines.

Hereinafter, there will be described the immunological characteristics of a specific heterodimer molecule formed by the fusion of the Par1 and Parj2 sequences and mutated in the respective positions 4, 29 and 30 (PjEDcys). In particular, such a protein was generated via the genetic fusion of the two polypeptides in the order Parj2/4,29,30-Par1/4,29,30. The fusion event caused the insertion of two additional amino acids (G and F) that do not interfere with the correct reading phase (Fig. 2 and 4, panel A). Such a fusion protein was produced and purified by using a commercial prokaryotic expression system (fusion protein expression system pQE30, QIAGEN). Fig. 4, panel B, shows a Western-blot analysis from which it is inferred that the mutated dimer protein exhibits a reduced allergenicity (lane D) if compared to the IgE binding activity of the individual monomers (lanes A and B) or to a dimer molecule consisting of the wild type Parj1 and Parj2 allergens (lane C). This demonstrates that both the fusion event and the introduced mutation contribute to the attainment of the described hypoallergenicity. This data cannot be ascribed to a different amount of protein loaded on the gel, as it is demonstrated by panel C, where a probe specific for histidine tails was used. The reduced binding capability was then demonstrated by a technique independent from the preceding one where the heterodimer molecule PjEDcys was assayed for its capability of inhibiting human IgE binding to a Parietaria crude extract. In fact, Fig. 5 shows how this molecule inhibits IgE binding of 5 allergic patients with a value ranging from 3.5 to 10% and anyhow with values extremely reduced with respect to a dimer construct containing the Parj1 and Parj2 allergens in their native form (Wt dimer clone).

The effect of reduced binding capability was then demonstrated in a histamine release assay carried out on peripheral blood of allergic patients. The data reported in Fig.6 show the histamine release percentages of the PjEDcys heterodimer as related to the release percentages of an equimolar mixture of the wild type Parj1 and Parj2 monomers. In all patients studied (N=4) the mutated molecule exhibits a marked reduction of the anaphylactic activity.

These variations at a structural level not only do not reduce the immunogenic capability of the molecule; on the contrary, they enhance its characteristics. In fact, in Fig. 7 it is reported the cell proliferation graph obtained by incubating PBMC purified from the blood of an allergic subject and after stimulation with the PjEDcys clone and with the mixture of wild type Parj1 and Parj 2 allergens. The mutations with regard to the cysteine residues and the fusion of the two proteins have no qualitative effect on the processing and the recognition of the allergen by blood T cells, yet they do markedly enhance the intensity thereof, as demonstrated by the Table (panel D) of the same Fig. 7.

Hereinafter, the invention will be illustrated by means of specific examples concerning the experimental steps of the preparation and the assessment of the immunological properties of the Parj2/4,29,30-Par1/4,29,30 fusion molecule. These examples have a merely illustrative purpose, in no way being limitative of the invention.

### Example 1: Construction of a molecule containing genetic information for the parj2 mutated in cys 4, 29 and 30 (Parj2/4,29,30 clone).

Site-specific mutagenesis with regard to cysteine residues in positions 29 and 30 was carried out using Transformer Site-Directed Mutagenesis kit (Clontech) following the manufacturer's instructions and using the synthetic oligonucleotide Pj2/29-30 5' GAG AGC AGC AGC GGC AGC 3'(SEQ ID NO 5). The clone, capable of expressing the wild type Parj2, was used as template for the mutagenesis and the cysteine residues in positions 29 and 30 were transformed into serine (Parj2/29-30 clone). Process success was confirmed by recombinant clone sequencing using the Sanger method. Mutagenesis of the cysteine residue in position 4 into serine was obtained by DNA polymerase chain reaction (PCR) using the synthetic oligonucleotides Pj2/4 5' GTG GGA TCC GAG GAG GCT AGC GGG AAA GTG 3' (SEQ ID NO 6) and Pj2 reverse 5' GGG GGA TCC ATA GTA ACC TCT GAA 3' (SEQ ID NO 7) and using the Parj2/29-30 clone as template. The DNA fragment thus obtained was cloned in the pQE30 expression vector (Qiagen). Analysis of the nucleotide sequence of the recombinant clone demonstrated substitution had occurred (see Fig.1) .

### Example 2: construction of a dimer molecule containing genetic information for the parj1 and parj2 mutated in cys 4, 29 and 30.

The dimer molecule consisting of the Par1 and Parj2 allergens mutated in positions Cys4, Cys29 and Cys30, respectively, was obtained by a series of DNA amplification processes.

The Parj1 clone mutated in the cysteines at positions Cys4, Cys29 and Cys30 disclosed in patent n. WO 02/20790 (clone 29-30) was digested with BamH1 restriction enzyme.

The fragment containing the genetic information for the Parj2 mutated in positions Cys4, 29 and 30 (Parj2/4,29,30 clone) was subjected to DNA amplification process using oligonucleotides Pj2/4 and Pj2 reverse. The fragment thus generated was purified by agarose gel, digested with BamH1 restriction enzyme and incubated with a mixture containing the enzyme DNA ligase and the Parj 1 (29-30) clone previously linearised. Recombinant clones were purified and their nucleotide sequence determined by Sanger method. The hybrid protein thus constructed was expressed as fusion protein with one histidine tail in its amino terminal region to allow the purification thereof (PjEDcys clone) on affinity column. (See Fig. 2 and Fig. 4, panel A).

### Example 3: Induction and purification of recombinant proteins.

10 ml O/N culture were used for an inoculation in 400 ml of 2YT culture medium containing ampicillin and kanamycin to a final concentration of 100 µg/ml and 10 µg/ml, respectively. The growth occurs at 37°C and under stirring. At +2 hour, IPTG to the final concentration of 1 mM was added to the culture and the growth proceeded for other 4 hours at 37°C under stirring. Then, the bacterial culture was centrifuged at 5000 rpm for 15 min at 4° C. Pellet was resuspended in 5 ml/g Start buffer (10mM Na phosphate pH7.4 and 6 M UREA) and the cells destroyed by using a sonicator. Then, recombinant proteins were definitively purified by using a His Trap column (Amersham) following the manufacturer's instructions. Eluted fractions were analysed on 16% polyacrylamide gel and fractions containing the recombinant protein were quantitatively assessed with Bradford method at the spectrophotometer after staining. Finally, proteins were desalted by using a Sephadex G-25 column (Pharmacia).

### Example 4: ELISA assay for assessing the percentage of inhibition of IgE binding.

ELISA test detection was carried out as described in Bonura et al. "Hypoallergenic variants of the Parietaria judaica major allergen Par j 1: a member of the non-specific lipid transfer protein plant family" Int Arch Allergy Immunol. 2001 Sep; 126 (1):32-40.), or as described in the abovementioned App. WO-A-02/020790. The concentration of the antigen used in each well is of 5 µg/ml. The patients tested (n=5) had a clear history of allergy to *Parietaria judaica,* and all tested positive to skin test using commercial products.

The results of the binding inhibition test with respect to the native protein and of the forms modified by elimination of two, three or four disulphide bridges are reported in the Table of Fig. 9.

The results observed with the Wt heterodimer and the PjEDcys heterodimer are reported in the Table of Fig. 5. Lastly, the results observed with the *Parietaria* crude extract, the individual Wt allergens and the mixture thereof are reported in the Table of Fig. 8.

### Example 5: histamine release assay

Histamine release assay was carried out using heparinised blood from Pj allergic patients and an allergen concentration scale ranging from 0.0001 and 1 µg/ml. Release protocol was carried out as previously described (Colombo, P., et al., Identification of an immunodominant IgE epitope of the Parietaria Judaica major allergen. J. Immunol, 1998. 160(6): p. 2780-5).

### Example 6: Study of PjEDcys-induced CD3+ cell proliferation

PBMC from Pj allergic patients were purified and resuspended in 1XPBS pH 7.2 (1x10⁷/ml) and labelled with Carboxy-Fluorescein Diacetate Succinimidyl Ester (CFDA-SE) to a final concentration of 5 mM for 5 min, at room temperature and in the dark. Cells were washed in complete RPMI (10% AB serum) and stimulated 7 days with a mixture containing 1 µg/ml Parj1 and Parj2 allergens and with an equimolar mixture of the PjEDcys hybrid. Then, the PBMC were stained with anti-CD3-PE antibody and analysed under cytofluorometry. The resulting data were analysed using the WinMDI 2.8 software. PBMC proliferation was determined by CFDA-SE (Carboxy-Fluorescein Diacetate Succinimidyl Ester) staining. Cytofluorometric analysis demonstrated that in the 5 subjects studied the PjEDcys hybrid is capable of stimulating a percentage of CD3+ cells far greater with respect to those stimulated by the mixture containing the allergens in monomer form (Fig. 7).

## Claims

1. A fusion protein **characterised in that** it comprises the amino acid sequences of the different allergens Parj1 and Parj2, belonging to the non-specific Lipid Transfer Protein (ns-LTPs) family from *Parietaria judaica* species, **in that** said sequences lack one or more of the four disulphide bridges present in the sequence of the wild type allergens, at least one in the amino terminal region comprised between the amino acid residues 1 and 30 and **in that** said sequences maintain essentially the same length of the sequences of wild type allergens.

2. The fusion protein according to claim 1, **characterised in that** the amino acid sequence of each of the allergens is independently mutated by elimination or substitution of one or more cysteine residues involved in the formation of a disulphide bridge.

3. The fusion protein according to any one of the claims 1 to 2, **characterised in that** the amino acid sequence of each of the allergens is independently mutated by elimination or substitution of one or more cysteine residues in positions corresponding to the positions 4, 14, 29, 30, 50, 52, 75 and 91 of the amino acid sequence of Parj1 and/or Parj2 allergen.

4. The fusion protein according to any one of the claims 1 to 3, **characterised in that** it contains the amino acid sequences of the Parj1 and Parj2 allergens, both independently modified by substitution of cysteine residues with Asn, Ser, Thr, Ile, Met, Gly, Ala, Val, Gln or Leu residues in positions 29 and 30 or 4, 29 and 30 or 29, 30, 50, 52.

5. The fusion protein according to claim 4, having the amino acid sequence SEQ ID NO: 4.

6. A nucleotide sequence comprising the DNA cording for the fusion protein according to any one of the claims 1 to 5.

7. The nucleotide sequence according to claim 6 comprising the nucleotide sequence SEQ ID NO: 3.

8. An expression or cloning system comprising the nucleotide sequence according to claims 6 or 7 flanked by suitable sequences for controlling, promoting and regulating the expression.

9. A host cell transformed by means of the expression or cloning system according to claim 8.

10. The fusion protein according to any one of the claims 1 to 5, for use in a diagnostic or therapeutic treatment method *in vivo* and/or *in vitro.*

11. The fusion protein according to claim 10, for use as hypoallergenic immunologic agent in the specific immunotherapy (SIT) treatment of allergies.

12. The fusion protein according to claim 10, for use in the treatment of rhinitis, conjunctivitis, urticaria, angioedema, eczema, dermatitides, asthma, anaphylactic shock.

13. The fusion protein according to claim 10 , for the preparation of DNA vaccines.

14. A pharmaceutical composition comprising the fusion protein according to any one of the claims 1 to 5 and a pharmaceutically acceptable excipient.

15. The pharmaceutical composition according to claim 14 in the form of solution, suspension, emulsion, cream, ointment or implant.

16. The pharmaceutical composition according to claim 14, for a parenteral, subcutaneous, intramuscular, intravenous, topical, oral administration or for subcutaneous implantation.

17. A method of preparation of the fusion protein according to any one of the claims 1 to 5, **characterised in that** suitably mutated amino acid sequences of different allergens are produced and linked directly or via a spacer for chemical synthesis or by expression, in the form of fusion protein, in genetically modified host cells.

18. The method of preparation according to claim 17, **characterised in that** host cells are transformed with an expression vector comprising the DNA coding for the amino acid sequences in fused form, mutated via site-specific mutagenesis in codons coding for cysteine residues.

19. The method of preparation according to claim 18, **characterised in that** one or more cysteine residues are substituted with Asn, Ser, Thr, Ile, Met, Gly, Ala, Val, GIn or Leu residues.

20. The method of preparation according to any one of the claims 17 to 19, **characterised in that** one or more cysteine residues in position 29, 30 or 4, 29, 30 or 29, 30, 50, 52 are substituted with alanine or serine residues.

21. The method of preparation of a pharmaceutical composition according to any one of the claims 14 to 16, **characterised in that** the heterodimer protein is mixed in an immunologically active amount to a pharmaceutically acceptable excipient.

## Patentansprüche

1. Fusionsprotein, **dadurch gekennzeichnet, dass** es die Aminosäuresequenzen der unterschiedlichen Allergene Parj1 und Parj2 umfasst, die zur Familie der nichtspezifischen Lipidtransferproteine (ns-LTPs) der Spezies *Parietaria judaica* gehören, **dadurch**, dass den Sequenzen eine oder mehrere der vier Disulfidbrücken fehlen, die in der Sequenz der Wildtypallergene anwesend sind, wobei mindestens eine in der aminoterminalen Region ist, die innerhalb der Aminosäurereste 1 bis 30 liegt, und **dadurch**, dass die Sequenzen im Wesentlichen die gleiche Länge wie die Sequenzen der Wildtypallergene beibehalten.

2. Fusionsprotein nach Anspruch 1, **dadurch gekennzeichnet, dass** die Aminosäuresequenz von jedem der Allergene unabhängig voneinander durch Eliminierung oder Substitution von einem oder mehreren Cysteinresten mutiert ist, die an der Bildung einer Disulfidbrücke beteiligt sind.

3. Fusionsprotein nach einem der Ansprüche 1 bis 2, **dadurch gekennzeichnet, dass** die Aminosäuresequenz von jedem der Allergene unabhängig voneinander durch Eliminierung oder Substitution von einem oder mehreren Cysteinresten an Positionen mutiert ist, die den Positionen 4, 14, 29, 30, 50, 52, 75 und 91 der Aminosäuresequenz des Parj1- und/oder Parj2-Allergens entsprechen.

4. Fusionsprotein nach einem der Ansprüche 1 bis 3, **dadurch gekennzeichnet, dass** es die Aminosäuresequenzen der Parj1- und Parj2-Allergene enthält, wobei beide unabhängig voneinander durch Substitution von Cysteinresten mit Asn, Ser, Thr, Ile, Met, Gly, Ala, Val, Gln oder Leu an den Positionen 29 und 30 oder 4, 29 und 30 oder 29, 30, 50, 52 modifiziert sind.

5. Fusionsprotein nach Anspruch 4, das die Aminosäuresequenz SEQ ID NO: 4 hat.

6. Nukleotidsequenz umfassend die DNA, die das Fusionsprotein nach einem der Ansprüche 1 bis 5 kodiert.

7. Nukleotidsequenz nach Anspruch 6, umfassend die Nukleotidsequenz SEQ ID NO: 3.

8. Expressions- oder Klonierungssystem, umfassend die Nukleotidsequenz nach Anspruch 6 oder 7, flankiert von geeigneten Sequenzen für das Kontrollieren, Fördern und Regulieren der Expression.

9. Wirtszelle, die mittels des Expressions- oder Klonierungssystems nach Anspruch 8 transformiert ist.

10. Fusionsprotein nach einem der Ansprüche 1 bis 5 zur Verwendung in einem diagnostischen oder therapeutischen Behandlungsverfahren *in vivo* und/oder *in vitro.*

11. Fusionsprotein nach Anspruch 10 zur Verwendung als hypoallergenes immunologisches Agens in der spezifischen Immuntherapie (SIT)-Behandlung von Allergien.

12. Fusionsprotein nach Anspruch 10 zur Verwendung in der Behandlung von Rhinitis, Konjunktivitis, Urticaria, Angioödem, Ekzem, Dermatitiden, Asthma, anaphylaktischem Schock.

13. Fusionsprotein nach Anspruch 10 für die Herstellung von DNA-Impfstoffen.

14. Pharmazeutische Zusammensetzung umfassend das Fusionsprotein nach einem der Ansprüche 1 bis 5 und einen pharmazeutisch verträglichen Exzipienten.

15. Pharmazeutische Zusammensetzung nach Anspruch 14 in Form einer Lösung, Suspension, Emulsion, Creme, Salbe oder eines Implantats.

16. Pharmazeutische Zusammensetzung nach Anspruch 14 für eine parenterale, subkutane, intramuskuläre, intravenöse, topische, orale Verabreichung oder für subkutane Implantation.

17. Verfahren zur Herstellung des Fusionsproteins nach einem der Ansprüche 1 bis 5, **dadurch gekennzeichnet, dass** Aminosäuresequenzen verschiedener Allergene hergestellt werden, die in geeigneter Weise mutiert sind und direkt oder mittels eines Spacers verbunden werden durch chemische Synthese oder mittels Expression in Form eines Fusionsproteins in genetisch modifizierten Wirtszellen.

18. Verfahren zur Herstellung nach Anspruch 17, **dadurch gekennzeichnet, dass** die Wirtszellen mit einem Expressionsvektor transformiert werden, der die DNA umfasst, die die Aminosäuresequenzen in fusionierter Form kodiert, wobei jene mittels ortsspezifischer Mutagenese in Codons mutiert ist, die Cysteinreste kodieren.

19. Verfahren zur Herstellung nach Anspruch 18, **dadurch gekennzeichnet, dass** einer oder mehrere Cysteinreste mit Asn-, Ser-, Thr-, Ile-, Met-, Gly-, Ala-, Val-, Gln- oder Leu-Resten substituiert sind.

20. Verfahren zur Herstellung nach einem der Ansprüche 17 bis 19, **dadurch gekennzeichnet, dass** einer oder mehrere Cysteinreste an Position 29, 30 oder 4, 29, 30 oder 29, 30, 50, 52 mit Alanin- oder Serinresten substituiert sind.

21. Verfahren zur Herstellung einer pharmazeutischen Zusammensetzung nach einem der Ansprüche 14 bis 16, **dadurch gekennzeichnet, dass** das heterodimere Protein in einer immunologisch aktiven Menge mit einem pharmazeutisch verträglichen Exzipienten vermischt wird.

## Revendications

1. Protéine de fusion **caractérisée en ce qu'**elle comprend les séquences d'acides aminés des différents allergènes Parj1 et Parj2, appartenant à la famille des protéines de transfert de lipides non spécifiques (ns-LTP) de l'espèce *Parietaria judaica,* **en ce que** lesdites séquences sont dépourvues d'un ou de plusieurs des quatre ponts disulfures présents dans la séquence des allergènes de type sauvage, au moins un étant dans la région amino-terminale comprise entre les résidus d'acides aminés 1 et 30, et **en ce que** lesdites séquences conservent essentiellement la même longueur que les séquences des allergènes de type sauvage.

2. Protéine de fusion selon la revendication 1, **caractérisée en ce que** la séquence d'acides aminés de chacun des allergènes est indépendamment mutée par une élimination ou une substitution d'un ou de plusieurs résidus cystéine impliqués dans la formation d'un pont disulfure.

3. Protéine de fusion selon l'une quelconque des revendications 1 à 2, **caractérisée en ce que** la séquence d'acides aminés de chacun des allergènes est indépendamment mutée par une élimination ou une substitution d'un ou de plusieurs résidus cystéine aux positions correspondant aux positions 4, 14, 29, 30, 50, 52, 75 et 91 de la séquence d'acides aminés d'un allergène Parj 1 et/ou Parj2.

4. Protéine de fusion selon l'une quelconque des revendications 1 à 3, **caractérisée en ce qu'**elle contient les séquences d'acides aminés des allergènes Parj1 et Parj2, toutes deux étant indépendamment modifiées par une substitution des résidus cystéine par Asn, Ser, Thr, Ile, Met, Gly, Ala, Val, Gln ou Leu aux positions 29 et 30 ou 4, 29 et 30 ou 29, 30, 50, 52.

5. Protéine de fusion selon la revendication 4, ayant la séquence d'acides aminés SEQ ID NO: 4.

6. Séquence de nucléotides comprenant l'ADN codant pour la protéine de fusion selon l'une quelconque des revendications 1 à 5.

7. Séquence de nucléotides selon la revendication 6, comprenant la séquence de nucléotides SEQ ID NO: 3.

8. Système d'expression ou de clonage comprenant la séquence de nucléotides selon les revendications 6 ou 7, flanquée par des séquences appropriées pour contrôler, promouvoir et réguler l'expression.

9. Cellule hôte transformée au moyen du système d'expression ou de clonage selon la revendication 8.

10. Protéine de fusion selon l'une quelconque des revendications 1 à 5, pour une utilisation dans un procédé de traitement diagnostique ou thérapeutique *in vivo* et/ou *in vitro.*

11. Protéine de fusion selon la revendication 10, pour une utilisation en tant qu'agent immunologique hypoallergénique dans le traitement des allergies par immunothérapie spécifique (ITS).

12. Protéine de fusion selon la revendication 10, pour une utilisation dans le traitement de la rhinite, de la conjonctivite, de l'urticaire, de l'angioedème, de l'eczéma, de la dermatite, de l'asthme, du choc anaphylactique.

13. Protéine de fusion selon la revendication 10, pour la préparation de vaccins à ADN.

14. Composition pharmaceutique comprenant la protéine de fusion selon l'une quelconque des revendications 1 à 5, et un excipient pharmaceutiquement acceptable.

15. Composition pharmaceutique selon la revendication 14, sous la forme d'une solution, d'une suspension, d'une émulsion, d'une crème, d'une pommade ou d'un implant.

16. Composition pharmaceutique selon la revendication 14, destinée à une administration parentérale, sous-cutanée, intramusculaire, intraveineuse, topique, orale, ou à une implantation sous-cutanée.

17. Procédé de préparation de la protéine de fusion selon l'une quelconque des revendications 1 à 5, **caractérisé en ce que** des séquences d'acides aminés de différents allergènes, mutées de manière appropriée, sont produites et sont liées directement ou via un espaceur, par une synthèse chimique ou par une expression, sous la forme d'une protéine de fusion, dans des cellules hôtes génétiquement modifiées.

18. Procédé de préparation selon la revendication 17, **caractérisé en ce que** des cellules hôtes sont transformées avec un vecteur d'expression comprenant l'ADN codant pour les séquences d'acides aminés sous une forme fusionnée, mutées via une mutagenèse site-spécifique dans des codons codant pour des résidus cystéine.

19. Procédé de préparation selon la revendication 18, **caractérisé en ce qu'**un ou plusieurs résidus cystéine sont substitués par des résidus Asn, Ser, Thr, Ile, Met, Gly, Ala, Val, Gln ou Leu.

20. Procédé de préparation selon l'une quelconque des revendications 17 à 19, **caractérisé en ce qu'**un ou plusieurs résidus cystéine à la position 29, 30 ou 4, 29, 30 ou 29, 30, 50, 52 sont substitués par des résidus alanine ou sérine.

21. Procédé de préparation d'une composition pharmaceutique selon l'une quelconque des revendications 14 à 16, **caractérisé en ce que** la protéine hétérodimère est mélangée en une quantité immunologiquement efficace à un excipient pharmaceutiquement acceptable.
